# FASCICULE DE BREVET EUROPEEN

(11) **EP 1 255 508 B1**
(45) Date de publication et mention de la délivrance du brevet: **23.08.2006**
(21) Numéro de dépôt: 01907772.6
(22) Date de dépôt: 13.02.2001
(51) Int. Cl.: A61F 2/16

(54) **IMPLANT INTRAOCULAIRE ET DISPOSITIF DE CRISTALLIN ARTIFICIEL**
INTRAOKULÄRES IMPLANTAT UND KÜNSTLICHES LINSENTEIL
INTRAOCULAR IMPLANT AND ARTIFICIAL CRYSTALLINE DEVICE

(30) Priorité: 16.02.2000 FR 0001884
(43) Date de publication de la demande: 13.11.2002
(73) Titulaire: HumanOptics AG, 91054 Erlangen (DE)
(72) Inventeur: HANNA, Khalil, F-75007 Paris (FR)
(74) Mandataire: Rau, Albrecht
(86) Numéro de dépôt international: PCT/FR2001/000407
(87) Numéro de publication internationale: WO 2001/060286

(56) Documents cités:
- WO-A1-99/03427
- WO-A1-99/29266
- FR-A1- 2 778 093

## Description

La présente invention concerne le domaine des implants intraoculaires et plus précisément celui des implants cristalliniens lors de l'opération de la cataracte ayant consisté à retirer la matière cristalline du sac capsulaire au travers d'une capsulotomie centrale et antérieure (capsulorexis) de 5 à 6 mm de diamètre. De ce fait la partie postérieure et la partie équatoriale du sac capsulaire sont préservées.

Le remplacement du cristallin naturel par un implant est devenu une opération courante dans le domaine de la chirurgie de la cataracte.

Jusqu'à présent, les implants mis en place n'ont pas le pouvoir de donner au patient opéré des facultés d'accommodation. Or on sait que la perte d'accommodation qui est la cause de la presbytie a pour origine non pas une dégradation du fonctionnement du muscle ciliaire et des fibres zonulaires qui lui sont associées, mais d'une part un durcissement de la matière cristalline contenue dans le sac capsulaire et d'autre part l'augmentation des dimensions du cristallin en raison de l'âge du patient. Le durcissement de la matière cristalline s'oppose à la modification de sa forme par le sac capsulaire lorsque celui-ci est relâché par les fibres zonulaires (vision de près). L'augmentation de la taille du cristallin a pour conséquence une transmission partielle seulement de l'amplitude de la variation de dimension du muscle ciliaire au sac capsulaire, une partie de cette amplitude consistant, lors de la relaxation du muscle, à tendre les fibres zonulaires avant d'en provoquer le déplacement vers l'extérieur générateur d'une modification de la forme de la lentille cristallinienne.

On sait que, chez un sujet jeune, la matière cristalline seule c'est-à-dire non enfermée dans le sac capsulaire a plutôt la forme du cristallin à l'état dé-accommodé (vision de loin). C'est dans cet état qu'avec l'âge la matière cristalline tend à durcir. Or, toujours chez un sujet jeune, la forme au repos du cristallin, c'est-à-dire de la matière cristalline dans le sac capsulaire et en l'absence de toute liaison avec les fibres zonulaires, est proche de celle à l'état accommodé (vision de près). En d'autres termes, le sac capsulaire par son élasticité contraint la matière cristalline à quitter son état de repos pour adopter une forme d'accommodation. Le durcissement s'oppose à ce moulage de la matière cristalline par le sac capsulaire.

De même chez un sujet jeune, les fibres zonulaires axiales sont toujours tendues sous contrainte à l'état accommodé de l'oeil. Elles permettent ainsi de transmettre au sac capsulaire toute l'amplitude de la déformation du muscle ciliaire. L'augmentation de taille du cristallin détend les fibres zonulaires axiales lorsque le muscle ciliaire est contracté si bien qu'une relaxation de ce muscle n'a d'effet sur le sac capsulaire que sur une partie de sa course, la première partie de cette augmentation de diamètre ayant pour seul effet de retendre les fibres zonulaires axiales, afin qu'elles redeviennent motrices pour le sac capsulaire dans une seconde partie de l'augmentation de diamètre du muscle ciliaire.

Ainsi lorsque la chirurgie de la cataracte respecte l'intégrité de la partie postérieure du sac capsulaire, de sa partie équatoriale et laisse subsister une portion périphérique de la paroi antérieure, toutes les conditions existent pour qu'une accommodation puisse être recouvrée. En effet, la pleine capacité de déformation élastique du sac capsulaire est retrouvée ; en l'absence de matière cristalline, le sac se rétrécit élastiquement et les fibres zonulaires recouvrent leur tension. Il est alors possible de bénéficier de "l'ensemble moteur" encore fonctionnel que constituent notamment le muscle ciliaire, les fibres zonulaires et les parties restantes du sac capsulaire.

De nombreux implants ont été conçus pour tenter d'exploiter la contraction-relaxation du muscle ciliaire afin de modifier la puissance optique de l'oeil. On connaît des implants qui possèdent deux pièces, à savoir une enveloppe logée dans le sac capsulaire et une partie optique à l'intérieur de l'enveloppe. L'enveloppe est censée suivre la forme du sac capsulaire. Il s'ensuit, au moins théoriquement, un déplacement le long de l'axe optique de l'oeil de la pièce optique qui entraîne une variation de la puissance optique de l'oeil donc une accommodation de la vision. On citera à cet égard le document EP O 337 390. Il apparaît que cet implant est peu performant car le mécanisme d'écrasement de l'enveloppe ne produit qu'un mouvement très faible de la partie optique, donc une faculté d'accommodation quasi inexistante.

On connaît également des implants en une seule pièce qui comportent une partie centrale optique et une partie haptique (par exemple deux bras radiaux implantés à la périphérie de la partie optique) dont la fonction est d'être emprisonnées dans la zone équatoriale du sac capsulaire qui collapse et par la formation d'une fibrose. La méthode opératoire consiste, après mise en place de l'implant entre les parties de membranes collapsées, à maintenir le muscle ciliaire à l'état relaxé le temps (quelques semaines) que la fibrose ait emprisonné les extrémités des parties haptiques. Pendant ce temps, le reste de la partie antérieure du sac capsulaire se rétrécit, ce qui tend à contraindre les parties haptiques vers la partie postérieure du sac et donc à appliquer la partie optique contre cette partie postérieure. Après la fin de la croissance fibrotique, le muscle ciliaire est autorisé à être normalement commandé par le cerveau. Ainsi, lorsqu'il se contracte, pour la vision de près, le sac capsulaire est relâché, et la zone fibrosée tend d'une part à basculer vers l'avant aidée en cela par une augmentation de la pression interne de l'oeil et d'autre part à se resserrer radialement, ce qui provoque un mouvement de la partie optique vers l'avant, cette rétraction radiale étant transformée par les bras haptiques articulés ou flexibles en un mouvement de protusion vers l'avant de la partie optique. Pour accomplir ce mouvement et le mouvement inverse lors d'une relaxation du muscle ciliaire, les parties haptiques sont articulées au bord de la partie optique ou très souples pour pouvoir passer ou fléchir devant et derrière le plan moyen de cette dernière, devant pour la vision de loin et derrière pour la vision de près. En outre les parties haptiques glissent dans les poches du tissu fibrosé qui a été engendré entre les parties collapsées du sac capsulaire au voisinage de son équateur. Ce type d'implant est par exemple décrit dans le document US 5 674 282.

Dans ce dispositif, le tissu fibrosé dont on favorise la croissance est un facteur de modification de l'interaction entre les fibres zonulaires et le sac capsulaire qui empêche de prédire le comportement final de l'implant lors de l'accommodation.

On a enfin proposé un autre implant accommodatif en une seule pièce qui comporte une partie annulaire dont la section est en forme de gouttière destinée à être logée dans la zone équatoriale du sac capsulaire et de laquelle partent des bras de liaison d'une pièce centrale optique. Les variations centripètes et centrifuges du diamètre de la zone équatoriale du sac entraînent une poussée ou une traction radiale sur les bras qui provoquent un déplacement de la pièce optique le long de l'axe optique (voir WO 99 03427)

Dans ce dispositif la présence de la pièce annulaire extérieure continue constitue un frein à la déformation de la zone équatoriale du sac qui diminue l'efficacité du pouvoir accommodatif de l'implant.

La présente invention permet, contrairement aux dispositifs connus de respecter au mieux les facultés encore disponibles de l'oeil opéré de la cataracte, pour accommoder.

Ceci s'effectue par le fait que l'extrémité libre de chaque bras radial est équipée de sa propre semelle par liaison du type charnière.

La présente invention a pour premier objet un implant en une seule pièce et pour second objet un dispositif de cristallin artificiel qui comporte avec l'implant une pièce intermédiaire entre l'implant et le sac capsulaire.

Dans la présente description il faut entendre antérieur et postérieur dans leur sens connu en ophtalmologie, c'est-à-dire antérieur étant pour le système cristallinien ce qui est le plus proche de la cornée, et postérieur ce qui est le plus éloigné de la cornée. Dans ce qui suit, on adoptera ces deux adjectifs même pour des dispositifs non implantés, leur description étant faite comme s'ils l'étaient.

Ainsi, l'implant selon l'invention est un implant accommodatif à loger dans le sac capsulaire comportant en une seule pièce en matériau élastiquement déformable une lentille centrale optique et au moins deux parties haptiques en forme de bras radiaux pour prendre appui par leur extrémité libre dans la zone équatoriale du sac capsulaire ; l'extrémité libre de chaque bras radial est équipée d'une semelle d'appui de l'implant sur la zone équatoriale du sac dont la surface extérieure est sensiblement conique, la liaison de la semelle au bras correspondant étant du type charnière située au voisinage du bord postérieur de la semelle par une première partie amincie du bras tandis que la liaison du bras à la lentille est du type charnière réalisée au niveau de la surface antérieure de la lentille par une seconde partie également amincie du bras, le plan contenant les premières parties amincies étant situé en arrière du plan contenant les secondes parties amincies.

Plusieurs avantages résultent de cette structure. Tout d'abord, tout mouvement tendant à rapprocher les semelles du centre de la lentille engendre un déplacement vers l'avant de cette lentille, ce qui correspond à une contraction du muscle ciliaire pour la vision de près.

Ce mouvement vers l'avant sera d'autant plus significatif que :
- les semelles transmettent intégralement la diminution de diamètre du sac capsulaire contrairement à un anneau équatorial qui offre toujours à vaincre une résistance à une contraction radiale,
- les semelles diminuent sensiblement la production de tissu fibrotique qui forme une masse au niveau de l'équateur du sac capsulaire modifiant (dans le sens d'une moins grande déformabilité) les caractéristiques de ce sac et donc son aptitude à répondre sur la plus grande amplitude possible aux variations de tension des fibres zonulaires. A ce propos on préférera des semelles circonférentiellement assez longues pour justement s'opposer à la croissance de la fibrose (au moins le tiers de la circonférence du sac).

De préférence, les bras possèdent une excroissance postérieure pour qu'à l'état le plus dilaté radialement, cette excroissance prenne appui sur la paroi postérieure du sac capsulaire et empêche une inversion des plans des charnières qui interdirait toute accommodation.

De préférence aussi, chaque bras est en forme d'arche avec le pied de chaque arche relié par une partie amincie à la lentille optique. On comprend que par cette géométrie on peut allonger la longueur utile de chaque bras haptique donc pour une contraction radiale donnée obtenir une plus grande amplitude du déplacement vers l'avant de la lentille. La longueur utile maximale de bras est celle du bras qui est articulé à la lentille au niveau du diamètre de celle-ci perpendiculaire au rayon médian du bras. Dans ces conditions cependant la liaison bras / lentille serait concentrée aux deux extrémités de ce diamètre et rendrait instable l'orientation de cette lentille. C'est pourquoi une forme préférée de réalisation sera un implant à trois bras haptiques répartis à 120° autour de la lentille.

On notera à ce propos que pour obtenir un déplacement maximal de la lentille, il faut s'assurer que l'écart entre les plans antérieur et postérieur contenant les deux types de charnières soit le plus petit possible pour profiter du domaine de variation maximale de la fonction sinusoïdale qui gouverne la transmission des mouvements.

Par ailleurs, l'implant selon l'invention comporte avantageusement, entre chaque bras haptique, des extensions radiales rigides enracinées à la périphérie de la lentille qui forment des butées s'opposant à l'expulsion de l'implant hors du sac capsulaire en venant au contact de la portion de paroi antérieure restante du sac bordant l'ouverture centrale qui y a été pratiquée. Ces extensions sont placées en dehors de la bissectrice de l'angle séparant deux bras haptiques de sorte que le pliage de l'implant reste aisé le long de certains diamètres de cet implant dépourvus et de bras et d'extensions.

Dans le cas où les bras affectent une forme d'arche, la liaison à charnière entre la lentille et chaque pied de bras est réalisée par l'intermédiaire de ces extensions radiales, juste à l'extérieur du diamètre maximum de la lentille optique.

Le second objet de l'invention est un dispositif de cristallin artificiel qui comporte, en plus de l'implant précédemment décrit, un élément intermédiaire élastiquement déformable notamment en direction radiale qui est destiné à revêtir la face intérieure du sac capsulaire ou au moins sa zone équatoriale. Donc, en plus de l'implant, ce dispositif comporte un élément séparé de l'implant, déformable élastiquement avec au moins une partie périphérique en forme de gouttière radialement déformable dont le diamètre en fond de gouttière, à l'état de repos, est inférieur au diamètre extérieur de l'implant mesuré sur la face externe de chaque semelle, à l'état de repos de celui-ci.

Le diamètre équatorial extérieur de cet élément intermédiaire, toujours dans son état de repos, correspond au diamètre équatorial du cristallin accommodé du sujet auquel il est destiné lorsque ce sujet avait entre 20 et 30 ans.

Lorsqu'on place l'implant dans la gouttière, on obtient un état d'équilibre de l'ensemble qui est tel que le diamètre équatorial extérieur de l'ensemble est supérieur à celui de l'élément intermédiaire seul et que le diamètre équatorial de l'implant est inférieur à celui qu'il avait au repos. Cet état d'équilibre est celui atteint lorsque les forces de contraction radiale de la pièce en gouttière sont égales aux forces d'extension radiale de l'implant.

Par des méthodes de calcul informatique il est possible de déterminer pour des caractéristiques mécaniques données (donc des matières données) les différentes géométries et dimensions critiques de l'implant et de la gouttière notamment au niveau des charnières des bras haptiques de l'implant et au niveau de l'épaisseur de la partie équatoriale de la gouttière qui conditionnent tel ou tel état d'équilibre et les quantités d'énergie nécessaires à sa modification. Il est alors possible d'adapter l'implant au sujet qui le reçoit et ainsi optimiser le pouvoir accommodatif de ce sujet.

Par exemple si la capsulorexis de la paroi antérieure est de petite taille et si la production de tissus fibrotique par le sujet est supposée peu importante, on mettra en place dans le sac capsulaire un dispositif dont l'état d'équilibre est proche de l'état accommodé de son cristallin naturel lorsqu'il avait 25 ou 30 ans. En revanche, avec une capsulorexis de grande taille et une tendance à la fibrose importante le dispositif mis en place aura un état dans lequel l'implant sera dans une position par rapport à l'élément intermédiaire proche de la vision de loin, la dimension extérieure du dispositif étant encore celle du cristallin naturel du sujet lorsqu'il avait 25 - 30 ans à l'état dé-accomodé (en l'absence d'accommodation).

Après la mise en place du dispositif dans le sac capsulaire, ce dernier tend à se contracter élastiquement jusqu'à entrer au contact de l'élément du dispositif qui forme l'enveloppe de l'implant. Comme la dimension de cette enveloppe correspond à celle de la matière cristalline lorsque le patient était jeune (entre 25 et 30 ans), c'est-à-dire à l'âge où le pouvoir accommodatif est important, tous les composants moteurs de l'accommodation (notamment les fibres zonulaires) se retrouvent dans leur état d'efficacité maximale telle qu'elle existait à l'époque.

D'autres caractéristiques et avantages ressortiront de la description donnée ci-après de quelques exemples de réalisation de l'invention.

Il sera fait référence aux dessins annexés parmi lesquels :
- la figure 1 est une vue de la face antérieure d'un implant selon l'invention,
- la figure 2 est une vue en coupe selon la ligne II-II de la figure 1,
- la figure 3 est une vue en coupe selon la ligne III-III de la figure 1,
- les figures 4 et 5 sont des vues en perspective respectivement par la face antérieure et par la face postérieure d'une variante de réalisation de l'implant de la figure 1,
- la figure 6 est une vue en perspective par la face postérieure d'une autre variante de réalisation de l'implant selon l'invention,
- la figure 7 est une vue en perspective de dessus d'un autre mode de réalisation de l'implant selon l'invention,
- la figure 8 illustre par une vue en perspective une première réalisation de la pièce en gouttière du dispositif de cristallin artificiel selon l'invention,
- la figure 9 est une même vue d'une variante de réalisation de cette pièce en gouttière,
- les figures 10 et 11 sont des représentations des sections des pièces respectivement de la figure 8 et de la figure 7 dans les plans IV-IV et V-V de ces figures,
- les figures 12 et 13 illustrent par des mêmes vues une variante de réalisation des figures 10 et 11,
- la figure 14 est une vue de dessus en plan d'une autre variante de réalisation de l'implant selon l'invention,
- les figures 15 et 16 illustrent par des sections diamétrales le dispositif selon les figures 7 et 9 respectivement dans son état de vision de près et dans son état de vision de loin,
- les figures 17 et 18 sont des figures identiques à celles des figures 15 et 16 pour le dispositif réalisé avec les pièces des figures 7 et 8 respectivement dans les mêmes états,
- la figure 19 est une vue en coupe d'un dispositif selon les figures 12 et 13 dans un état d'équilibre proche d'un état de vision de loin,
- les figures 20, 21 et 22 illustrent une autre variante de réalisation du dispositif selon l'invention.

La matière dont sont constitués les différents composants de l'implant selon l'invention est une matière bien connue dans le domaine en question et par exemple acrylique pliable, élastomère de silicone. Ces matières sont déformables élastiquement de manière d'une part à pouvoir être repliées sur elles-mêmes pour introduire l'implant dans le sac capsulaire au travers d'une incision de petite taille réalisée dans l'oeil du patient et, d'autre part, comportant une mémoire de forme dont l'effet tend à placer chacune de ces pièces dans sa forme de repos.

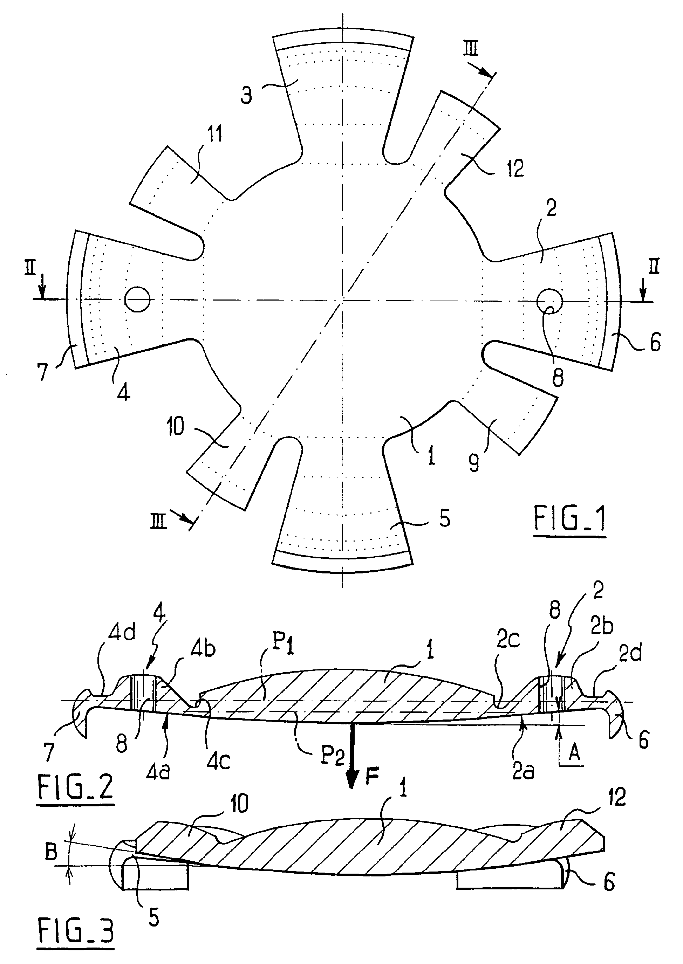

L'implant représenté aux figures 1, 2 et 3 comporte une lentille centrale 1 par exemple biconvexe de diamètre de l'ordre de 5 à 6 millimètres. A la périphérie de cette lentille centrale 1, l'implant comporte quatre bras haptiques 2, 3, 4 et 5 s'étendant radialement vers l'extérieur et vers l'arrière de la lentille 1. A titre indicatif, pour illustrer cette inclinaison vers l'arrière des bras haptiques, la surface antérieure 2a, 4a de chaque bras forme avec le plan médian de la lentille un angle A compris entre 2° et 15° (de préférence 5°).

On remarquera sur les figures 2 et 3 que chaque bras possède une section médiane 2b, 4b renflée sur sa face postérieure située entre une partie mince 2c, 4c de raccordement à la périphérie de la lentille et une partie mince 2d, 4d de raccordement du bras à une semelle 6, 7 qui est sensiblement perpendiculaire au plan général de la lentille et dont la surface extérieure est sensiblement torique. La semelle de chaque bras haptique s'étend majoritairement en saillie de la surface antérieure 2a, 4a du bras correspondant. En d'autres termes les zones amincies de liaison 2d, 4d sont voisines du bord postérieur des semelles. Les zones amincies 2c, 2d, 4c, 4d sont réalisées par des gorges sur la face postérieure des bras et la zone 2c, 4c sera de préférence plus mince que la zone 2d, 4d (par exemple respectivement 70 et 200 microns pour une épaisseur de la lentille de 1 millimètre et une matière - acrylique - donnée). Au moins l'un des quatre bras haptiques (deux sur les figures) est pourvu d'un orifice 8 qui permet au chirurgien une manipulation de l'implant à l'intérieur du sac capsulaire à l'aide d'un outil introduit dans cet orifice.

Les parties amincies 2c, 2d, 4c, 4d constituent les charnières entre chaque bras et d'une part la lentille et d'autre part la semelle. L'axe de rotation de ces charnières est considéré comme étant approximativement situé au milieu de l'épaisseur de chaque zone. Le plan P1 contenant chaque charnière du type 2d, 4d est situé à l'arrière du plan P2 contenant les charnières du type 2c, 4c de manière qu'une compression radiale appliquée sur les semelles engendre un mouvement de la lentille 1 dans le sens de la flèche F.

L'implant représenté à ces figures comporte, intercalées entre les bras haptiques 2-5 des extensions radiales 9, 10, 11, 12 représentées en coupe à la figure 3. Ces extensions rigides constituent des organes qui viennent buter contre le reste de la paroi antérieure du sac capsulaire qui entoure l'ouverture pratiquée dans cette paroi. Afin que cette butée n'intervienne pas lors d'un mouvement relevant d'un phénomène normal d'accommodation, la dimension radiale de ces extensions est inférieure à celle des bras haptiques et leur extension est inclinée vers la face postérieure de l'implant d'un angle B compris entre 10° et 20°. L'implantation angulaire de ces extensions n'est pas sur la bissectrice de l'angle formé par deux bras haptiques adjacents. Cette caractéristique permet de laisser deux zones au voisinage de ces bissectrices, dépourvues de bras ou d'extensions ce qui rend le pliage de l'implant plus aisé autour des diamètres passant par ces zones.

La longueur angulaire de chaque bras haptique au niveau de la semelle est dans le cas des figures 1 à 3 de l'ordre de 120° au total. On pourra prévoir des valeurs plus importantes pour une meilleure transmission des forces et pour contenir une fibrose tendant à collapser les deux parois du sac capsulaire au niveau de l'équateur.

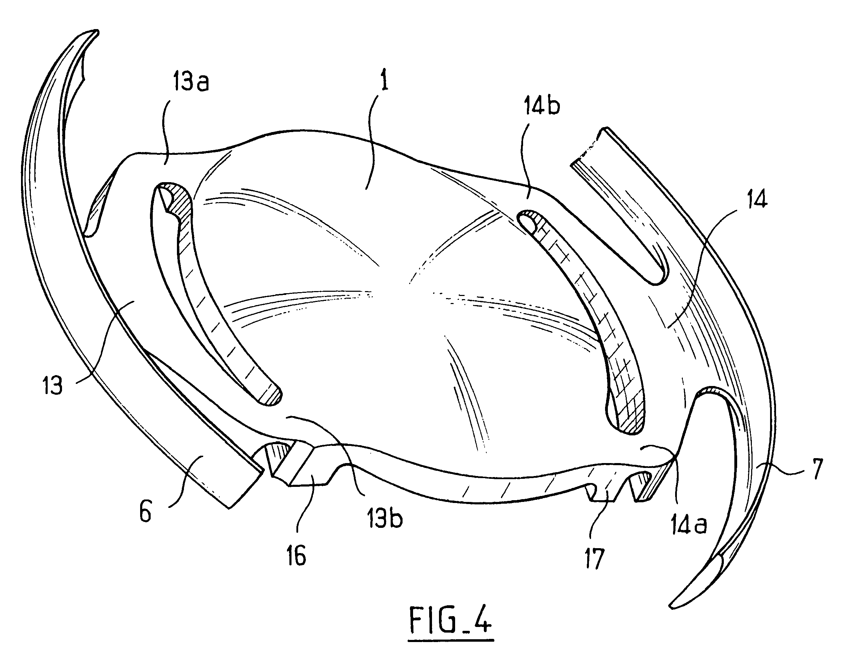

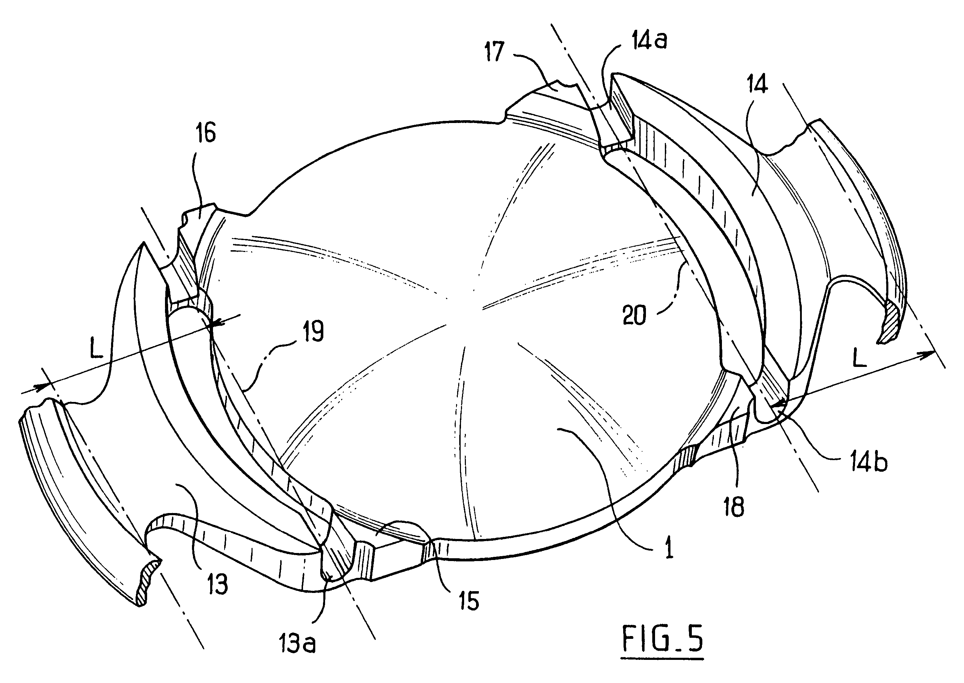

La variante de réalisation de l'implant représentée aux figures 4 (face antérieure) et figure 5 (face postérieure) comporte deux bras haptiques 13 et 14 en forme d'arche équipés à l'extérieur d'une semelle 6, 7 (ici représentée) d'une longueur angulaire importante (entre 60° et 90°) et raccordés à la lentille par l'intermédiaire de pieds 13a, 13b, 14a, 14b prenant racine dans des extensions radiales 15, 16, 17, 18, ici de forme triangulaires, qui constituent les butées décrites ci-dessus. Les pieds 13a, 13b, 14a, et 14b sont amincis chacun par une gorge, les gorges concernant un bras sont d'axe commun 19, 20 définissant l'axe de la charnière de liaison du bras à la lentille. On comprend qu'en faisant varier la position des pieds 13a, 13b, et 14a, 14b le long de la lentille, on fait varier la longueur L utile de chaque bras, longueur séparant la charnière de la semelle au bras de la charnière du bras à la lentille. On cherchera à trouver un compromis entre une plus grande longueur utile facteur d'un déplacement important le long de l'axe optique et la stabilité du maintien de la lentille par les bras.

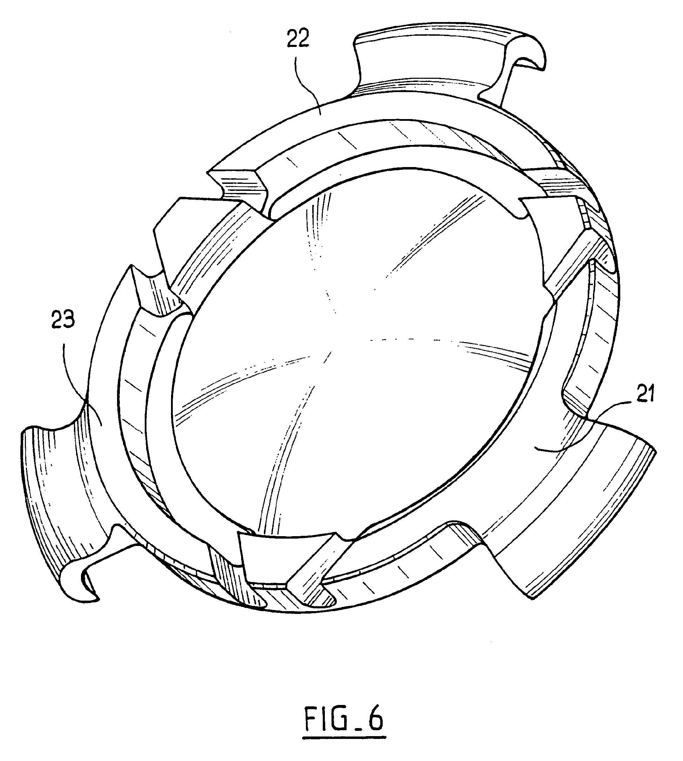

Alors qu'aux figures 4 et 5 les pieds des bras en arche sont implantés à 90° l'un de l'autre dans la variante représentée à la figure 6, les bras haptiques 21, 22, 23 sont au nombre de 3 avec des pieds pour chaque bras espacés d'environ 120°. Toutes choses égales par ailleurs, la longueur utile de chaque bras est dans cette variante plus grande que dans celle des figures 4 et 5.

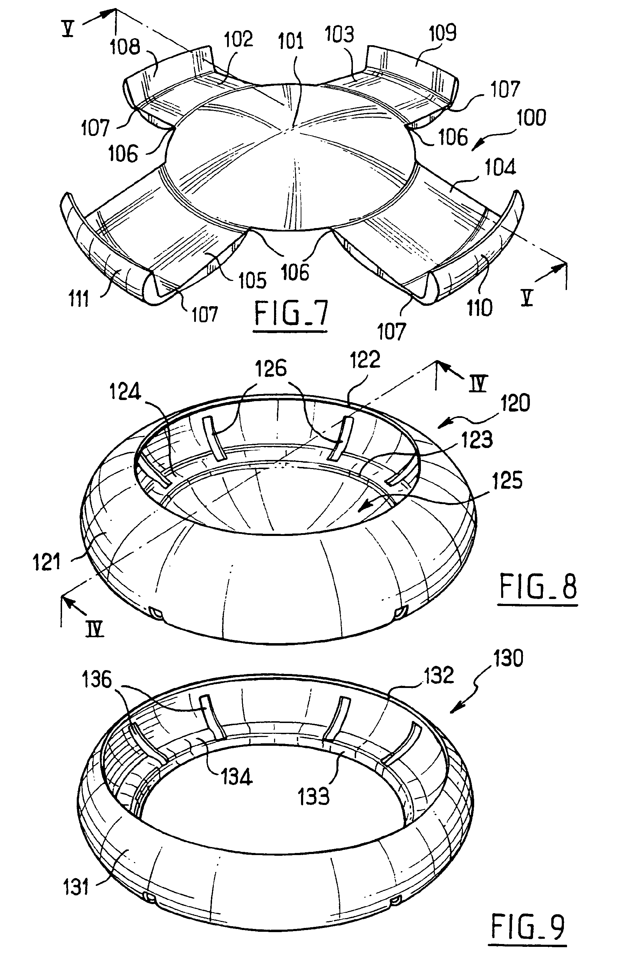

Le dispositif de cristallin artificiel décrit en regard des figures 7 à 19 comporte un implant qui peut être identique à celui décrit précédemment (ou dans la forme représentée à ces figures) et un élément séparé qui vient revêtir au moins la surface intérieure équatoriale du sac capsulaire.

Comme dans les figures précédentes, l'implant 100 comporte une lentille centrale 101 par exemple biconvexe de diamètre de l'ordre de 5 à 6 mm et quatre bras haptiques 102, 103, 104 et 105 s'étendant radialement vers l'extérieur et vers l'arrière de la lentille 101 (la face visible sur la figure 7 est la face avant de cette pièce).

Chaque bras possède une section radiale renflée entre une partie mince 106 de raccordement à la périphérie de la lentille 101 et une partie mince 107 de raccordement du bras à une semelle 108, 109, 110, 111 comme dans les figures précédentes.

Les parties 106 et 107 constituent des charnières élastiques des bras d'une part à la lentille centrale et d'autre part aux semelles. On notera que dans un tel implant destiné à coopérer avec l'élément séparé, l'épaisseur des zones formant charnières sera en général, pour une matière donnée, plus importante que celle des charnières d'un implant utilisé seul.

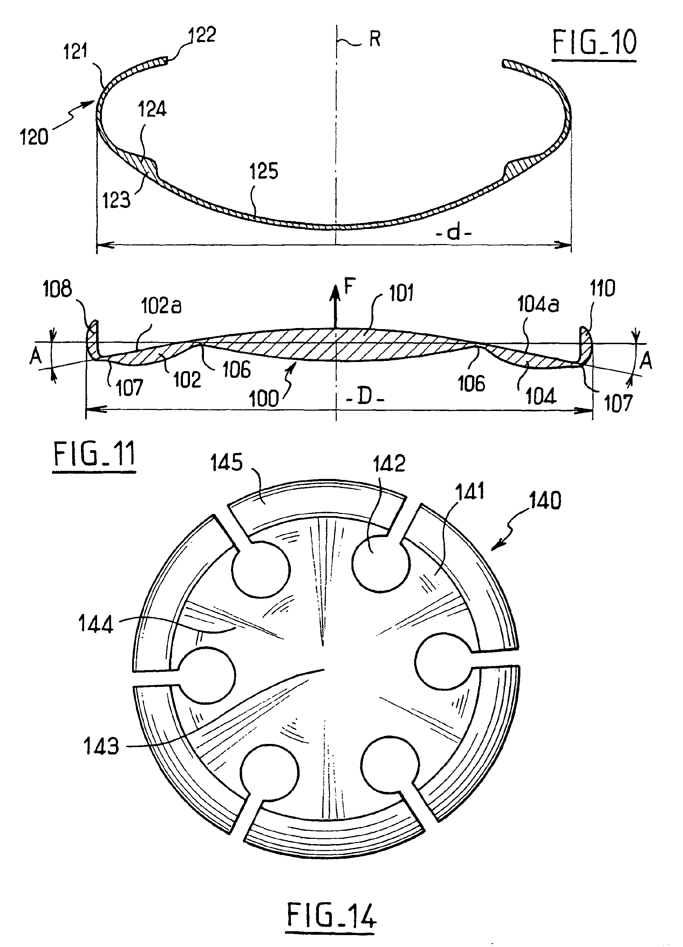

L'implant est représenté aux figures 7 et 11 dans son état de repos. On a noté D le diamètre de l'implant au repos mesuré à partir des surfaces extérieures de deux semelles opposées. A titre indicatif, on indiquera que cette dimension est comprise entre 9,5 mm et 10,5 mm.

Le dispositif selon l'invention comporte un élément séparé de l'implant représenté aux figures 8 et 10 dans son état de repos, selon une première réalisation et à la figure 9 selon une autre réalisation. Cet élément 120 porte une partie en forme de gouttière 121 limitée par une lèvre antérieure 122 et une lèvre postérieure 123 pourvue d'un bourrelet intérieur 124, la lèvre postérieure 123 se prolongeant en un dôme 125 refermant la partie postérieure de cet élément 120. Cette partie en gouttière est pourvue de fentes transversales 126 coopérant à son pouvoir de déformation tant dans une direction parallèle à son axe de révolution R que dans une direction perpendiculaire à celui-ci.

Le plus grand diamètre noté d à la figure 10 de l'élément 120 appelé son diamètre équatorial, est au repos sensiblement égal au diamètre du cristallin d'un sujet, et notamment du sujet qui doit être opéré, lorsque ce dernier avait entre 25 et 30 ans et mesuré à l'état accommodé, c'est-à-dire pour la vision de près.

La variante 130 de l'élément 120 représentée à la figure 9 est identique à celle-ci à l'exception du dôme 125 qui n'existe plus sur l'élément 130. Cet élément se résume donc à une gouttière 131 avec des lèvres 132, 133 et un bourrelet 134 identique à la partie en gouttière 121 des figures 8 et 10.

Dans la variante représentée aux figures 12 et 13 l'élément séparé est du type de celui 130 de la figure 9 dans lequel les ouvertures 136 sont plus importantes pour autoriser une plus grande souplesse à la déformation de la gouttière et dont la lèvre inférieure 133b est dépourvue de bourrelet mais festonnée. Cet élément est adapté à recevoir un implant de la forme de celui représenté aux figures 1 à 3 et reproduit en figure 13. On constate comme dans le cas des figures 10 et 11 que le diamètre extérieur d de l'élément 130 (donc a fortiori son diamètre en fond de gouttière) est inférieur au diamètre D de l'implant.

La figure 14 est une vue de dessus d'une variante de réalisation 140 de l'implant 100 de la figure 7. La différence entre les deux réalisations tient au fait qu'à figure 14, l'implant 140 comporte six bras haptiques 141 séparés les uns des autres par un espace 142 en forme de trou de serrure. Ces bras sont, comme à la figure 7, reliés à la lentille optique centrale 143 par des zones charnières 144 et à une semelle d'extrémité 145 par également des zones charnières non visibles sur cette figure 14.

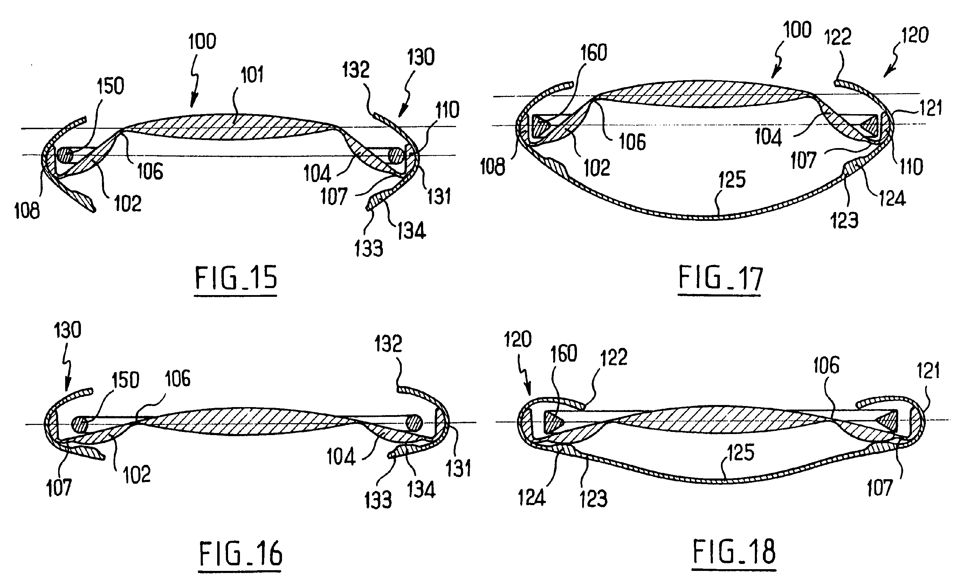

Aux figures 15 et 17, on a représenté le dispositif de cristallin artificiel selon l'invention dans son état d'équilibre lorsque l'implant 100 est logé dans l'élément enveloppe 120, 130. L'implant prend appui sur la surface intérieure de la gouttière au moyen de la surface extérieure sensiblement torique des semelles qui ainsi épousent le profil de cette surface intérieure. L'implant exerce une force sur l'élément enveloppe qui tend à le dilater radialement. Cet élément enveloppe oppose cependant à cette force une force qui conduit à un état de repos tel que l'implant est déformé beaucoup plus que ne l'est l'élément enveloppe. L'état d'équilibre du dispositif est donc son état pour vision de près (placé dans le sac capsulaire lorsque le muscle ciliaire est contracté, la forme de l'implant est voisine de cet état d'équilibre, le sac capsulaire n'exerçant pratiquement aucun effort sur la pièce enveloppe). La lentille optique est alors projetée en avant du plan équatorial de l'élément 120, 130.

Lorsque ce dispositif est placé dans le sac capsulaire, la relaxation du muscle ciliaire tend à le faire passer à l'état représenté aux figures 16 et 18. En effet, la relaxation du muscle ciliaire provoque une tension des fibres zonulaires qui, par l'intermédiaire du sac capsulaire, étire vers l'extérieur l'élément 120, 130 en aplatissant la gouttière, laissant alors à l'implant 100 la faculté de revenir à sa position de repos donc à ramener la lentille optique 101 au voisinage du plan équatorial de l'élément 120, 130. La limite du mouvement de la lentille optique vers l'arrière est atteinte lorsque les bras haptiques viennent au contact du bourrelet 124, 134 porté par la lèvre postérieure 123, 133 de l'élément 120, 130. On aura à cet égard noté que la déformation de cet élément entre les figures 15 et 17 et les figures 16 et 18 est non seulement une extension radiale de son diamètre équatorial mais également un rabattement des lèvres de la partie en gouttière l'une vers l'autre.

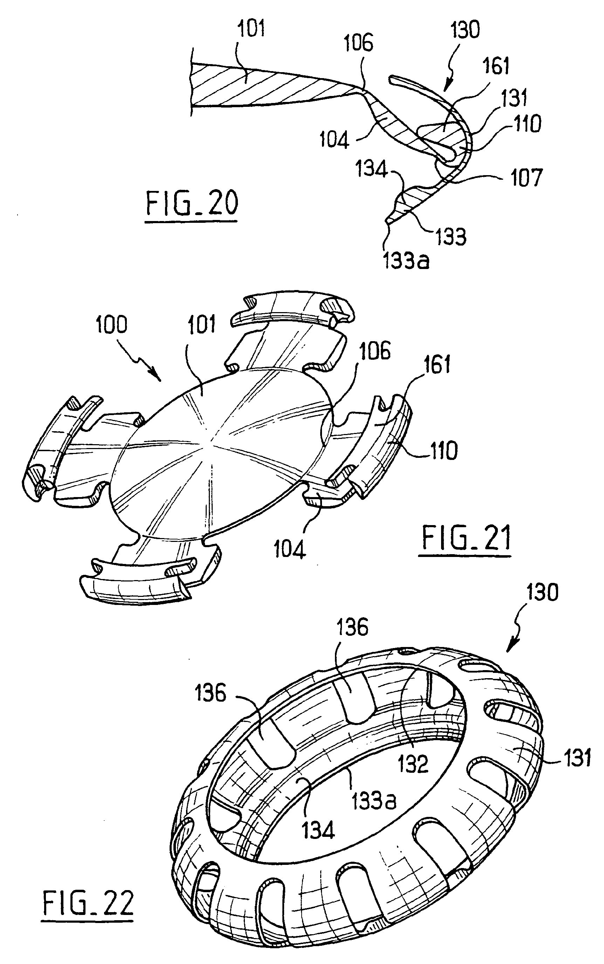

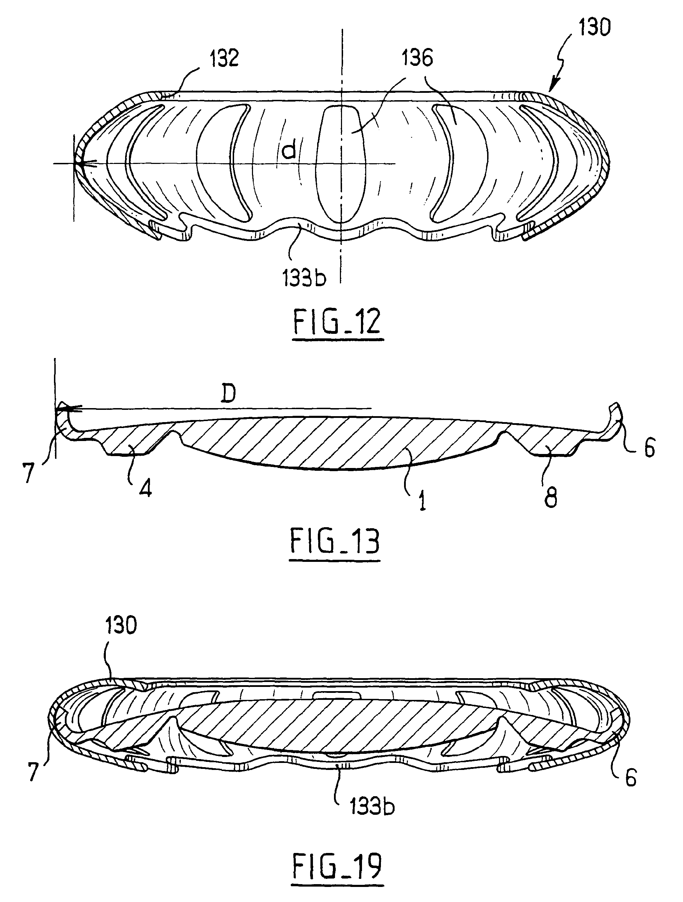

Le rôle de la membrane qui forme le sac capsulaire est pratiquement négligeable sur la pièce enveloppe de l'implant pour ce qui concerne sa capacité à se déformer. En revanche, le sac capsulaire constitue un moyen de transmission des forces exercées par les fibres zonulaires. Il se peut cependant qu'entre le sac capsulaire (qui par hypothèse est plus vaste que la pièce enveloppe de l'implant) et la pièce enveloppe de l'implant, après l'intervention, on ait à redouter qu'une fibrose se développe sur la face intérieure du sac. Dans cette perspective clinique on préférera alors mettre en place un dispositif tel que celui représenté à la figure 19 qui présente un état d'équilibre proche de l'état dé-accommodé (vision de loin) de l'oeil. Si cette fibrose prend des proportions anormales, on pourrait assister à une compression de l'élément 130 tendant à expulser l'implant hors du sac capsulaire. Pour contrer cette tendance, on rappellera l'existence des butées décrites en regard des figures 1 à 6. On peut enfin prévoir un dispositif selon l'invention tel que la dimension D de l'implant soit juste égale à la dimension d de l'élément en gouttière diminuée de son épaisseur (deux fois) dans la zone équatoriale de sorte que l'état d'équilibre de l'implant dans la gouttière corresponde à l'état de repos et de l'implant et de la gouttière. Dans ce cas, la gouttière sera de préférence déformable sous de très faibles efforts et jouera le rôle unique d'un doublage interne du sac capsulaire si celui-ci avait à être renforcé.

Dans une autre variante, le dispositif selon l'invention comporte une dernière pièce constituée par un anneau 150 de section circulaire aux figures 15 et 16 ou 160 de section triangulaire aux figures 17 et 18, lequel anneau est monté flottant à l'intérieur du dispositif de manière à venir au contact des semelles des bras haptiques afin de s'opposer vigoureusement à un excès de contraction radiale de l'élément gouttière. On voit donc aux figures 15 et 17 que cet anneau 150, 160 a une circonférence extérieure dont le diamètre est sensiblement égal au diamètre du dispositif dans son état d'équilibre mesuré sur la face interne des semelles des bras haptiques.

La figure 20 est une vue en coupe partielle d'une variante de réalisation du dispositif comme représenté à la figure 15. La différence entre ces réalisations tient d'une part au remplacement de l'anneau 150 par une excroissance interne 161 de la semelle 110 dont la fonction est identique à celle de l'anneau 150 (ou 160 comme à la figure 17), à savoir de s'opposer à un excès de contraction radiale de la gouttière. D'autre part cette différence tient également à la conformation de l'extrémité de la lèvre postérieure 133 de l'élément en gouttière 30 qui présente un becquet 133a formant une barrière contre la prolifération des cellules du sac capsulaire depuis la région équatoriale de ce dernier en direction du centre de ce sac.

Les figures 21 et 22 sont deux vues en perspective de cette variante de réalisation de l'invention dans des représentations semblables à celles respectivement des figures 7 et 10 avec des références identiques pour des éléments identiques.

On comprend que le dispositif selon l'invention se comporte comme un corps élastique qui peut être déformé de manière importante sous l'effet de forces extrêmement faibles (de l'ordre de 1 à 2 x 10⁻⁵ N). En effet les forces nécessaires à sa déformation ne sont que des forces additionnelles produites par le muscle ciliaire qui viennent rompre un état d'équilibre entre des forces antagonistes développées par l'implant sur l'élément-enveloppe et par cet élément sur l'implant, qui elles sont d'un autre ordre.

## Revendications

1. Implant intraoculaire accommodatif à loger dans le sac capsulaire comportant en une seule pièce en matériau élastiquement déformable une lentille centrale optique (1) et au moins deux parties haptiques (2, 4) en forme de bras radiaux pour prendre appui par leur extrémité libre dans la zone équatoriale du sac capsulaire, l'extrémité libre de chaque bras radial (2, 4) étant équipée de sa propre semelle d'appui (6, 7)de l'implant sur la zone équatoriale du sac dont la surface extérieure est sensiblement torique, la liaison de la semelle (6, 7) au bras correspondant (2, 4) étant du type charnière située au voisinage du bord postérieur de la semelle (6, 7) par une première partie amincie (2d, 4d) du bras tandis que la liaison du bras à la lentille est du type charnière réalisée au niveau de la surface antérieure de la lentille par une seconde partie (2c, 4c) également amincie du bras, le plan (P1) contenant les premières parties amincies étant situé en arrière du plan (P2) contenant les secondes parties amincies.

2. Implant selon la revendication 1, **caractérisé en ce que** chaque bras (2, 4) comporte entre lesdites parties amincies formant charnière un renflement postérieur (2b, 4b).

3. Implant selon l'une quelconque des revendications précédentes, **caractérisé en ce que** chaque bras (13, 14) est en forme d'arche dont les pieds (13a, 13b, 14a, 14b) sont raccordés à la lentille optique (1) par des zones amincies orientées le long d'une ligne perpendiculaire (19, 20) au rayon médian du bras.

4. Implant selon l'une quelconque des revendications précédentes, **caractérisé en ce qu'**il comprend à partir de la lentille (1) au moins deux extensions radiales (10, 12) rigides intercalées entre les bras haptiques de longueur radiale inférieure à celle des bras pour former des butées de l'implant contre les bords de l'ouverture du sac capsulaire.

5. Implant selon la revendication 4, **caractérisé en ce que** ces extensions-butées sont situés en dehors des bissectrices des angles séparant deux bras radiaux consécutifs.

6. Implant selon la revendication 3 et la revendication 4, **caractérisé en ce que** chaque extension radiale forme la zone commune (15, 16, 17, 18) de raccordement à la lentille de deux pieds adjacents de deux bras en forme d'arche.

7. Implant selon les revendications 4 et 6, **caractérisé en ce qu'**il comprend trois bras (21, 22, 23) en forme d'arche régulièrement disposés autour de la lentille (1).

8. Implant selon l'une quelconque des revendications précédentes, **caractérisé en ce que** la longueur angulaire du contact entre l'ensemble des semelles et la partie équatoriale du sac est au moins égale à 120°.

9. Dispositif de cristallin artificiel accommodatif comprenant un implant (100) selon l'une des revendications 1 à 8, **caractérisé en ce qu'**il comporte un élément (120, 130) séparé de l'implant, déformable élastiquement avec au moins une partie périphérique en forme de gouttière radialement déformable dont le diamètre en fond de gouttière, à l'état de repos est au plus égal au diamètre extérieur de l'implant mesuré sur la face externe de chaque semelle (110), à l'état de repos de celui-ci.

10. Dispositif selon la revendication 9, **caractérisé en ce que** le diamètre équatorial de l'élément (120, 130) séparé, mesuré à l'extérieur de la gouttière dans son état de repos, correspond au diamètre équatorial du sac capsulaire du patient auquel le dispositif est destiné, lorsque ce patient avait entre 20 et 30 ans.

11. Dispositif selon la revendication 9, **caractérisé en ce que** l'élément (120) susdit est en forme de dôme avec un voile (125) postérieur dont le bord est en forme de gouttière.

12. Dispositif selon la revendication 9, **caractérisé en ce que** l'élément séparé (130) susdit est limité à un anneau en gouttière dont la lèvre postérieure (133b) est festonnée.

13. Dispositif selon l'une quelconque des revendications 9 à 12, **caractérisé en ce que** la partie en forme de gouttière comporte une pluralité de fentes traversantes (126, 136).

14. Dispositif selon l'une quelconque des revendications 9 à 13, **caractérisé en ce que** la lèvre antérieure (122, 132) de la partie de gouttière est de rayon inférieur à la dimension radiale des extensions-butées de l'implant.

15. Dispositif selon l'une quelconque des revendications 9 à 12, **caractérisé en ce que** l'élément séparé (120, 130) comporte au moins en regard de l'extrémité de chaque bras de l'implant voisine de la semelle une surépaisseur (124, 134) formant butée d'appui postérieur du bras correspondant.

16. Dispositif selon l'une quelconque des revendications 9 à 15, **caractérisé en ce qu'**il comporte une troisième pièce (150, 160) formée par un anneau dont le plus grand diamètre est au plus égal au diamètre intérieur de la gouttière diminué de l'épaisseur des semelles des bras de l'implant mesuré lorsque le dispositif est dans son état d'équilibre.

## Claims

1. Accommodative intraocular implant for reception in the capsular bag, including in a single piece of a resiliently deformable material a central optical lens (1) and at least two haptic portions (2, 4) in form of radial arms for abutting in the equatorial zone of the capsular bag via their free ends, wherein the free end of each radial arm (2, 4) is provided with its own web (6, 7) for supporting the implant in the equatorial zone of the bag, the outer surface of which is substantially toric, wherein the connection of the web (6, 7) to the corresponding arm (2, 4) is of the type of a hinge located in the vicinity of the posterior edge of the web (6, 7) and formed by a first thinned portion (2d, 4d) of the arm, while the connection of the arm to the lens is of the type of hinge realized at the level of the anterior surface of the lens by a second, also thinned portion (2c, 4c) of the arm, wherein the plane (P1) containing the first thinned portions is located behind the plane (P2) containing the second thinned portions.

2. Implant according to claim 1, **characterized in that** each arm (2, 4) includes a posterior bulge (2b, 4b) between the thinned portions constituting a hinge.

3. Implant according to any one of the preceding claims, **characterized in that** each arm (13, 14) is in form of an arc, the legs (13a, 13b, 14a, 14b) of which are connected to the optical lens (1) by thinned zones oriented along a line (19, 20) perpendicular to the median radius of the arm.

4. Implant according to any one of the preceding claims, **characterized in that**, starting from the lens (1), it includes at least two rigid radial extensions (10, 12) interposed between the haptic arms, the extensions having a radial length, which is less than that of the arms in order to form abutments of the implant against the borders of the aperture of the capsular bag.

5. Implant according to claim 4, **characterized in that** these abutment extensions are located outside of the bisectors of the angles separating two consecutive radial arms.

6. Implant according to claim 3 and claim 4, **characterized in that** each radial extension constitutes the common zone (15, 16, 17, 18) for connection of two adjacent legs of two arched arms to the lens.

7. Implant according to claims 4 and 6, **characterized in that** it includes three arched arms (21, 22, 23) regularly disposed around the lens (1).

8. Implant according to any one of the preceding claims, **characterized in that** the angular length of contact between the entirety of the flanges and the equatorial portion of the bag is at least equal to 120°.

9. Accommodative artificial lens device including an implant (100) according to any one of claims 1 to 8, **characterized in that** it includes a resiliently deformable element (120, 130) separated from the implant, the element (120, 130) having at least one peripheral part in form of a radially deformable channel, the diameter of which on the bottom of the channel in a rest state is at most equal to the outer diameter of the implant, measured on the outer surface of each web (110), in the rest state thereof.

10. Device according to claim 9, **characterized in that** the equatorial diameter of the separated element (120, 130), measured on the outside of the channel in its rest state, corresponds to the equatorial diameter of the capsular bag of the patient, to which the device is intended, when this patient would be between 20 and 30 years old.

11. Device according to claim 9, **characterized in that** the above mentioned element (120) is in form of a dome with a posterior cover (125), the border of which is in channel shape.

12. Device according to claim 9, **characterized in that** the above mentioned separated element (130) is limited to a channel-shaped annulus, the posterior lip (133b) of which is bent.

13. Device according to any one of claims 9 to 12, **characterized in that** the channel-shaped part includes a plurality of passing slots (126, 136).

14. Device according to any one of claims 9 to 13, **characterized in that** the anterior lip (122, 132) of the channel part has a radius, which is less than the radial dimension of the abutment extensions of the implant.

15. Device according to any one of claims 9 to 12, **characterized in that** the separated element (120, 130) includes an excess thickness (124, 134) at least compared with the end of each arm of the implant adjacent to the web, which constitutes a posterior supporting abutment of the corresponding arm.

16. Device according to any one of claims 9 to 15, **characterized in that** it includes a third piece (150, 160) formed of an annulus, the greater diameter of which is at most equal to the inner diameter of the channel, reduced by the thickness of the webs of the arms of the implant, measured with the device in its balanced state.

## Patentansprüche

1. Akkommodierbares intraokulares Implantat zur Aufnahme im Kapselsack, das in einem einzigen Stück aus einem elastisch verformbaren Material eine zentrale optische Linse (1) und mindestens zwei Haptik-Teile (2, 4) in Form von radialen Armen zum Anliegen in der Äquatorialzone des Kapselsacks mit ihren freien Enden umfasst, wobei das freie Ende von jedem radialen Arm (2, 4) mit seinem eigenen Steg (6, 7) zur Abstützung des Implantats auf der Äquatorialzone des Sacks ausgestattet ist, dessen äußere Fläche im Wesentlichen torisch ist, wobei die Verbindung des Stegs (6, 7) mit dem entsprechenden Arm (2, 4) vom Typ eines Scharniers ist, das sich in der Nähe des posterioren Randes des Stegs (6, 7) befindet und durch einen ersten verjüngten Teil (2d, 4d) des Arms gebildet ist, während die Verbindung des Arms mit der Linse vom Typ eines Scharniers ist, das auf dem Niveau der anterioren Fläche der Linse durch einen zweiten ebenfalls verjüngten Teil (2c, 4c) des Arms realisiert ist, wobei die Ebene (P1), die die ersten verjüngten Teile enthält, hinter der Ebene (P2) liegt, die die zweiten verjüngten Teile enthält.

2. Implantat nach Anspruch 1, **dadurch gekennzeichnet, dass** jeder Arm (2, 4) zwischen den verjüngten Teilen, die ein Scharnier bilden, eine posteriore Verdickung (2b, 4b) aufweist.

3. Implantat nach einem der vorangehenden Ansprüche, **dadurch gekennzeichnet, dass** jeder Arm (13, 14) in Form eines Bogens vorliegt, dessen Füße (13a, 13b, 14a, 14b) mit der optischen Linse (1) durch verjüngte Zonen verbunden sind, die längs einer zum mittleren Radius des Arms senkrechten Linie (19, 20) orientiert sind.

4. Implantat nach einem der vorangehenden Ansprüche, **dadurch gekennzeichnet, dass** es ausgehend von der Linse (1) mindestens zwei radiale starre Erweiterungen (10, 12), die zwischen die Haptik-Arme eingefügt sind, mit einer radialen Länge aufweist, die geringer ist als jene der Arme, um Anschläge des Implantats an den Rändern der Öffnung des Kapselsacks zu bilden.

5. Implantat nach Anspruch 4, **dadurch gekennzeichnet, dass** diese Anschlag-Erweiterungen außerhalb der Halbierenden der Winkel liegen, die zwei aufeinander folgende radiale Arme trennen.

6. Implantat nach Anspruch 3 und Anspruch 4, **dadurch gekennzeichnet, dass** jede radiale Erweiterung die gemeinsame Zone (15, 16, 17, 18) zur Verbindung von zwei benachbarten Beinen von zwei bogenförmigen Armen mit der Linse bildet.

7. Implantat nach den Ansprüchen 4 und 6, **dadurch gekennzeichnet, dass** es drei bogenförmige Arme (21, 22, 23) umfasst, die regelmäßig um die Linse (1) angeordnet sind.

8. Implantat nach einem der vorangehenden Ansprüche, **dadurch gekennzeichnet, dass** die angulare Länge des Kontakts zwischen der Gesamtheit der Stege und dem Äquatorialteil des Sacks mindestens gleich 120° ist.

9. Akkommodierbare künstliche Linsenvorrichtung mit einem Implantat (100) nach einem der Ansprüche 1 bis 8, **dadurch gekennzeichnet, dass** sie ein von dem Implantat getrenntes, elastisch verformbares Element (120, 130) mit mindestens einem Umfangsteil in Form einer radial verformbaren Rinne umfasst, deren Durchmesser am Boden der Rinne im Ruhezustand höchstens gleich dem Außendurchmesser des Implantats, gemessen an der Außenfläche von jedem Steg (110), im Ruhezustand von diesem ist.

10. Vorrichtung nach Anspruch 9, **dadurch gekennzeichnet, dass** der Äquatorialdurchmesser des getrennten Elements (120, 130), an der Außenseite der Rinne in ihrem Ruhezustand gemessen, dem Äquatorialdurchmesser des Kapselsacks des Patienten entspricht, für den die Vorrichtung vorgesehen ist, wenn dieser Patient zwischen 20 und 30 Jahre alt wäre.

11. Vorrichtung nach Anspruch 9, **dadurch gekennzeichnet, dass** das vorstehend genannte Element (120) in Form einer Haube mit einer posterioren Umhüllung (125) vorliegt, deren Rand eine Rinnenform aufweist.

12. Vorrichtung nach Anspruch 9, **dadurch gekennzeichnet, dass** das vorstehend genannte getrennte Element (130) auf einen rinnenförmigen Ring begrenzt ist, dessen posteriore Lippe (133b) gebogen ist.

13. Vorrichtung nach einem der Ansprüche 9 bis 12, **dadurch gekennzeichnet, dass** der rinnenförmige Teil eine Vielzahl von Durchgangsschlitzen (126, 136) umfasst.

14. Vorrichtung nach einem der Ansprüche 9 bis 13, **dadurch gekennzeichnet, dass** die anteriore Lippe (122, 132) des Rinnenteils einen Radius aufweist, der kleiner ist als die radiale Abmessung der Anschlag-Erweiterungen des Implantats.

15. Vorrichtung nach einem der Ansprüche 9 bis 12, **dadurch gekennzeichnet, dass** das getrennte Element (120, 130) zumindest gegenüber dem Ende von jedem Arm des Implantats, das zum Steg benachbart ist, eine Überdicke (124, 134) aufweist, die einen posterioren Anlageanschlag des entsprechenden Arms bildet.

16. Vorrichtung nach einem der Ansprüche 9 bis 15, **dadurch gekennzeichnet, dass** sie ein drittes Stück (150, 160) umfasst, das aus einem Ring gebildet ist, dessen größerer Durchmesser höchstens gleich dem Innendurchmesser der Rinne, vermindert um die Dicke der Stege der Arme des Implantats, gemessen, wenn sich die Vorrichtung in ihrem Gleichgewichtszustand befindet, ist.
